# EUROPÄISCHE PATENTANMELDUNG

(11) **EP 3 582 227 A1**
(43) Veröffentlichungstag der Anmeldung: **18.12.2019**
(21) Anmeldenummer: 18178021.4
(22) Anmeldetag: 15.06.2018
(51) Int. Cl.: G16H 30/40, G16H 40/63

(54) **VERFAHREN ZUM BETRIEB EINER MEDIZINISCHEN BILDAUFNAHMEEINRICHTUNG, BILDAUFNAHMEEINRICHTUNG, COMPUTERPROGRAMM UND ELEKTRONISCH LESBARER DATENTRÄGER**

(71) Anmelder: Siemens Healthcare GmbH, 91052 Erlangen (DE)
(72) Erfinder: Baruth, Oliver, 91054 Erlangen (DE); Bernhardt, Philipp, 91301 Forchheim (DE); Berting, Andreas, 36381 Schlüchtern (DE); Böhm, Stefan, 90522 Oberasbach (DE); Leiblein, Rudolf, 91085 Weisendorf (DE); Lendl, Markus, 91242 Ottensoos (DE); Obler, Richard, 91058 Erlangen (DE); Spahn, Martin, 91054 Erlangen (DE); Stowasser, Boris, 91056 Erlangen (DE); Tashenov, Stanislav, 91336 Heroldsbach (DE)

(57) **Zusammenfassung**

Verfahren zum Betrieb einer medizinischen Bildaufnahmeeinrichtung (8) im Rahmen eines einem Bildaufnahmeziel dienenden Bildaufnahmevorgangs an einem Patienten (14), wobei die Steuerung der Bildaufnahmeeinrichtung (8) auf Grundlage von durch eine Steuereinrichtung (15) der Bildaufnahmeeinrichtung (8) umzusetzenden Betriebsparametern erfolgt, wobei die Betriebsparameter mittels eines Ermittlungsalgorithmus (6) vollständig automatisch aus wenigstens den Patienten (14) in Form eines Patientenmodells und/oder das Bildaufnahmeziel beschreibenden Eingangsdaten (2, 3, 4, 5) und einer Registrierung (1) des Patienten (14) mit einem Koordinatensystem der Bildaufnahmeeinrichtung (8) ermittelt und zur Steuerung der Bildaufnahmeeinrichtung (8) verwendet werden.

## Beschreibung

Die Erfindung betrifft ein Verfahren zum Betrieb einer medizinischen Bildaufnahmeeinrichtung im Rahmen eines einem Bildaufnahmeziel dienenden Bildaufnahmevorgangs an einem Patienten, wobei die Steuerung der Bildaufnahmeeinrichtung auf Grundlage von durch eine Steuereinrichtung der Bildaufnahmeeinrichtung umzusetzenden Betriebsparametern erfolgt. Daneben betrifft die Erfindung eine medizinische Bildaufnahmeeinrichtung, ein Computerprogramm und einen elektronisch lesbaren Datenträger.

Moderne medizinische Bildaufnahmeeinrichtungen stellen komplexe Systeme dar, deren Bedienung Benutzer vor große Herausforderungen stellt, um eine geeignete oder gar optimale Bildqualität bei einem Bildaufnahmevorgang an einem Patienten zu erzielen. Daher ist es im Stand der Technik bekannt, Benutzern manuell anwählbare, vordefinierte Messprotokolle zur Auswahl anzubieten, so dass diese aufgrund des Bildaufnahmeziels des aktuellen Bildaufnahmevorgangs ein passendes Messprotokoll auswählen können. Derartige Messprotokolle werden, insbesondere bei Röntgeneinrichtungen als Bildaufnahmeeinrichtungen, auch als "Organprogramme" bezeichnet.

Die Messprotokolle, die durch eine Steuereinrichtung der Bildaufnahmeeinrichtung umzusetzende Betriebsparameter definieren, sind dabei wie die möglichen Bildaufnahmeziele und medizinischen Disziplinen äußerst unterschiedlich. Beispielsweise können Messprotokolle für sowohl unterschiedliche diagnostische Untersuchungen als auch Interventionen im Bereich der Kardangiographie, Elektrophysiologie, Neurointervention, allgemeinen Angiographie, Pädiatrie, Chirurgie, usw., an unterschiedlichen Organen, beispielsweise Herz, Leber, Kopf, Beine, Spinalbereich, etc., angeboten werden. Die Messprotokolle unterscheiden sich in ihren Betriebsparametern, beispielsweise physikalischen Betriebsparametern, wie bei Röntgeneinrichtungen Dosis, Röhrenspannung, Pulslänge, Bildfrequenz, Detektorauflösung, Detektormodus, Röhrenfokuswahl und dergleichen, und/oder auf die Auswertung bezogenen Betriebsparametern wie Nativmodus, Subtraktionsmodus, allgemeine Bildüberlagerung (beispielsweise Fluoroskopie- und 3D-Bilddatensatz), Art und Weise der bildbasierten oder temporalen Rauschunterdrückung, frequenzabhängige Kontrastanhebungen und/oder Kontrastreduzierungen, Management der Bilddynamik, Bildschärfe und dergleichen. Insgesamt kann also gesagt werden, dass auf einer Bildaufnahmeeinrichtung mit einer steigenden Zahl der Einsatzmöglichkeiten und Einsatzgebiete eine immer größer werdende Zahl von optimierten Messprotokollen vorliegt, die geeignete Betriebsparameter, seien es Bildaufnahmeparameter oder Bildauswertungsparameter, bereitstellen.

Für einen jeweiligen Bildaufnahmevorgang, der auch einen Untersuchungsabschnitt und/oder Behandlungsabschnitt bilden kann, beispielsweise einen diagnostischen Anteil eines Behandlungsvorgangs, eine Platzierung von medizinischen Instrumenten wie Ballon, Stent, Spule, Katheter zur Ablation von Embolisationsmaterialien oder zur Gabe von chemotherapeutischen Materialien, Biopsienadel und dergleichen, wählt der Benutzer, beispielsweise ein Arzt, der die Diagnose/Therapie durchführt, ein entsprechendes, seiner Ansicht nach geeignetes Messprotokoll aus.

In der täglichen Praxis kann es häufig dazu kommen, dass das für den Bildaufnahmevorgang optimale Messprotokoll, insbesondere Organprogramm, nicht verwendet wird, nachdem beispielsweise der Arzt als Benutzer seinen Fokus und seine Konzentration auf die Untersuchung/Therapie und den Patienten legt oder sich trotz einer Schulung nicht bewusst ist, dass ein alternatives Messprotokoll eventuell bessere Bildaufnahmeergebnisse liefern würde. Zur Verbesserung wurde diesbezüglich bereits vorgeschlagen, Messprotokolle zu einer Abfolge für eine Gesamtuntersuchung bzw. eine Gesamtbehandlung zusammenzufassen und so eine definierte Folge von Messprotokollen für verschiedene Untersuchungs- bzw. Behandlungsabschnitte bereitzustellen. Eine solche Zusammenfassung von Messprotokollen löst jedoch nicht das Problem der großen Menge bereitstehender, manuell zu wählender Messprotokolle.

Der Erfindung liegt daher die Aufgabe zugrunde, eine die Bedienung einer Bildaufnahmeeinrichtung vereinfachende und zur Verbesserung der Bildaufnahmequalität beitragende Möglichkeit zur Steuerung des Betriebs einer Bildaufnahmeeinrichtung anzugeben.

Diese Aufgabe wird gelöst durch ein Verfahren mit den Merkmalen des Anspruchs 1, eine Bildaufnahmeeinrichtung mit den Merkmalen des Anspruchs 15, ein Computerprogramm mit den Merkmalen des Anspruchs 16 und einen elektronisch lesbaren Datenträger mit den Merkmalen des Anspruchs 17. Vorteilhafte Ausgestaltungen ergeben sich aus den Unteransprüchen.

Ein erfindungsgemäßes Verfahren zum Betrieb einer medizinischen Bildaufnahmeeinrichtung sieht vor, dass die Betriebsparameter mittels eines Ermittlungsalgorithmus vollständig automatisch aus wenigstens den Patienten in Form eines Patientenmodells und/oder das Bildaufnahmeziel beschreibenden Eingangsdaten und einer Registrierung des Patienten mit einem Koordinatensystem der Bildaufnahmeeinrichtung ermittelt und zur Steuerung der Bildaufnahmeeinrichtung verwendet werden.

Damit wird eine radikale Abwendung vom bekannten Stand der Technik, der verschiedene Messprotokolle bereitstellt, die manuell ausgewählt werden, angestrebt, indem gar keine Messprotokolle mehr in der Bildaufnahmeeinrichtung definiert werden, die entweder rein manuell oder systemseitig unterstützt angewählt werden, sondern es wird mithilfe einer systeminhärenten Intelligenz gemäß dem Ermittlungsalgorithmus unmittelbar Einfluss auf die physikalischen und bildverarbeitungstechnischen Betriebsparameter genommen, die für den jeweiligen Bildaufnahmevorgang, insbesondere als Untersuchungs- und/oder Behandlungsabschnitt, optimal sind. Mit anderen Worten schlägt die Erfindung vor, dass ein entsprechend befähigter Ermittlungsalgorithmus große Teile oder sogar die komplette bildrelevante Parametrierung vornimmt und somit die Benutzerinteraktion, beispielsweise in Form einer Messprotokollanwahl, minimiert oder gänzlich ausgeschlossen wird.

Ein solcher Ermittlungsalgorithmus, der im Übrigen nicht zwangsläufig, aber in einem bevorzugten Ausführungsbeispiel künstliche Intelligent umfassend gewählt werden kann, nutzt dabei insbesondere den Patienten in Form eines Patientenmodells beschreibende Eingangsdaten und eine vorliegende Registrierung des Patienten mit einem Koordinatensystem der Bildaufnahmeeinrichtung aus, das bedeutet, aufgrund der Registrierung ist auch die Lage des Patientenmodells zur Bildaufnahmeeinrichtung bekannt, so dass insbesondere darauf geschlossen werden kann, welcher Untersuchungsbereich aufgenommen werden soll, was wiederum deutliche Hinweise auf das Bildaufnahmeziel gibt. Insbesondere bezüglich des Bildaufnahmeziels können auch weitere Eingangsdaten, wie noch dargelegt werden wird, vorliegen, um gemeinsam möglichst genau schließen zu können, welche Betriebsparameter das beste Bildgebungsergebnis für die Bildgebungsart der Bildaufnahmeeinrichtung liefern. Weitere Eingangsparameter können, allgemein gesagt, System- und Umgebungsbedingungen betreffen, insbesondere verwendete medizinische Instrumente und/oder Hilfsmittel für den Bildaufnahmevorgang. Unter Berücksichtigung der Lage des Patienten und somit seiner anatomischen Strukturen zur Bildaufnahmeeinrichtung bzw. der entsprechenden Aufnahmeanordnung der Bildaufnahmeeinrichtung wird im Übrigen auch eine ständig aktualisierbare Informationsgrundlage geschaffen, die bei dynamisch wechselnden Untersuchungsbereichen Betriebsparameter geeignet nachführen kann, insbesondere bei der Nachverfolgung eines medizinischen Instruments, beispielsweise eines Katheters, durch den Körper eines Patienten, welcher beispielsweise vom Arm oder aber von der Leiste aus an den Zielort geführt werden soll, wobei bereits diese unterschiedlichen Eintrittsorte klar unterschiedliche geeignete Betriebsparameter nahelegen, die automatisch anhand der Eingangsdaten und der vorliegenden Registrierung durch den Ermittlungsalgorithmus ermittelt und in der Steuereinrichtung verwendet werden können.

Mit besonderem Vorteil können die Betriebsparameter neben Bildaufnahmeparametern (physikalischen Betriebsparametern) auch wenigstens einen Bildauswertungsparameter (bildverarbeitungstechnischer Parameter) umfassen. Nachdem viele (Vor-) Auswertungsschritte bezüglich des Bildaufnahmeergebnisses, also der Bilddaten, bereits innerhalb der Bildaufnahmeeinrichtung durchgeführt werden, ist es mithin besonders zweckmäßig, auch die Bildverarbeitung bereits im Hinblick auf das durch die Eingangsdaten beschriebene und somit aus diesen zumindest ableitbare Bildaufnahmeziel derart anzupassen, dass auch diesbezüglich eine verbesserte Bildqualität entsteht, ohne dass entsprechende manuelle Eingaben eines Benutzers benötigt werden. Mit besonderem Vorteil umfassen die automatisch mittels des Ermittlungsalgorithmus bestimmbaren Betriebsparameter mithin alle bislang durch Messprotokolle bereitgestellten bzw. bereitstellbare Betriebsparameter, so dass der Ermittlungsalgorithmus aufgrund der Eingangsdaten ein individuell auf den aktuellen Bildaufnahmevorgang und den aktuellen Patienten angepasstes Messprogramm automatisch zusammenstellen kann.

Der Ermittlungsalgorithmus nutzt mithin wenigstens teilweise explizit bzw. wenigstens teilweise implizit, beispielsweise durch künstliche Intelligenz, Entscheidungskriterien, die die Eingangsdaten bzw. daraus abgeleitete Zwischendaten auswerten. Ein für die vorliegende Erfindung wesentliches Entscheidungskriterium ist dabei die Nutzung der durch die Eingangsdaten gegebenen Informationen zur Ermittlung des im Sichtfeld der Bildaufnahmeeinrichtung befindlichen und interessierenden Organs (OOI - Organ of Interest), um hierauf basierend die optimalen physikalischen und insbesondere auch bildverarbeitungstechnischen Betriebsparameter automatisch anzuwählen.

Eine zweckmäßige Weiterbildung der vorliegenden Erfindung sieht vor, dass der Ermittlungsalgorithmus wenigstens einen Algorithmus der künstlichen Intelligenz umfasst, der durch maschinelles Lernen trainiert wurde, insbesondere mittels Deep Learning. Künstliche Intelligenz hat sich ein nicht zwangsläufig erforderliches, aber äußerst nützliches und die Gesamtqualität der automatisch ermittelten Betriebsparameter verbesserndes Werkzeug im Rahmen der vorliegenden Erfindung erwiesen. Gerade im Zusammenhang mit der steigenden Komplexität von Bildaufnahmeeinrichtungen, insbesondere aufgrund steigender Ausstattung und/oder steigender Anzahl der Einstellmöglichkeiten, bietet sich die künstliche Intelligenz an, Zusammenhänge zwischen Eingangsparametern und Betriebsparametern sowie gegebenenfalls auch Betriebsparametern und Betriebsparametern zu extrahieren und nach einem entsprechenden Lernvorgang zur weiteren Verbesserung der Qualität des Bildaufnahmeergebnisses zu nutzen. Hierfür werden geeignete Trainingsdaten herangezogen.

In diesem Zusammenhang sieht eine besonders zweckmäßige Ausgestaltung der vorliegenden Erfindung vor, dass Trainingsdaten für den Algorithmus der künstlichen Intelligenz durch Protokollierung einer insbesondere wenigstens eine Einzelparameteranpassung umfassenden Tätigkeit eines Benutzers bei der Verwendung von vordefinierten Messprotokollen mit vordefinierten Betriebsparametern ermittelt werden. Das bedeutet, es wird vorgeschlagen, bei gängigen, Messprotokolle, die benutzerseitig angepasst werden können, einsetzenden Bildaufnahmeeinrichtungen Auswahl- und Anpassungstätigkeiten von Benutzern, insbesondere auch unter Berücksichtigung der Qualität des Bildaufnahmeergebnisses, zu protokollieren, um somit Optimierungspotential in diesen Messprotokollen aufzufinden und einen deutlichen Vorteil gegenüber einer lediglich automatischen Anwahl geeigneter Messprotokolle erzielen zu können. Dabei werden zweckmäßig parallel auch die Eingangsdaten ermittelt und mitprotokolliert, insbesondere das Patientenmodell, so dass in den Trainingsdaten den ermittelten Eingangsdaten zugeordnete Ausgangsdaten, nämlich die Benutzereinstellungen, zugeordnet sind.

Wie bereits erwähnt, ist es ein besonders zweckmäßiges Merkmal der vorliegenden Erfindung, ein Patientenmodell zu verwenden, welches aufgrund der vorliegenden Registrierung und der Anpassung auf den aktuellen Patienten möglichst genau beschreibt, welcher Anteil des Patienten im Sichtfeld der Bildaufnahmeeinrichtung platziert ist. In diesem Zusammenhang sieht eine konkrete Ausgestaltung vor, dass als den Patienten beschreibende Patientendaten wenigstens das auf den aktuellen Patienten angepasste Patientenmodell verwendet wird, welches lokal aufgelöst die Zusammensetzung des Patienten beschreibt. Dabei kann zweckmäßigerweise als Patientenmodell ein computertomographiebasiertes Patientenmodell und/oder ein vektorbasiertes Patientenmodell und/oder ein die Lage wenigstens eines Teils der Organe und/oder den Verlauf von Blutgefäßen des Patienten abbildendes Patientenmodell verwendet werden. Das bedeutet, denkbar ist beispielsweise ein computertomographiebasiertes bzw. computertomographieartiges Patientenmodell, das alle wichtigen Organe und den Blutgefäßbaum des Patienten abbildet. Ein derartiges computertomographiebasiertes Patientenmodell kann beispielsweis aus einer Computertomographieaufnahme des aktuellen Patienten selbst abgeleitet werden, jedoch auch als generisches Modell herangezogen werden und eignet sich besonders bei einer Ausgestaltung der Bildaufnahmeeinrichtung als Röntgeneinrichtung, da es dann implizit auch die Abbildbarkeit entsprechender anatomischer Strukturen durch die Röntgenbildgebung beschreibt. Denkbar ist es jedoch auch, ein vektorbasiertes, einfaches Patientenmodell heranzuziehen, dass die Umrisse eines generischen Patienten und die Lage aller wichtigen Organe, auf die Umrisse bezogen, abbildet und, bevorzugt mittels der Registrierung, auf den aktuellen Patienten angepasst wird. Insgesamt sind mithin unterschiedlichste Ausgestaltungen von Patientenmodellen denkbar, wobei in bevorzugten Ausgestaltungen grundsätzlich das Patientenmodell in der Lage ist, gemeinsam mit der Registrierung wenigstens die Lage wichtiger Organe relativ zu der Bildaufnahmeeinrichtung bzw. deren Aufnahmeanordnung abzubilden.

Die Anpassung des Patientenmodells auf den aktuellen Patienten, falls es sich um ein generisches Patientenmodell handelt, kann beispielsweise über sonstige Patientendaten erfolgen, welche beispielsweise aus der Registrierung und/oder aus Vorabaufnahmen und/oder aus sonstigen Patienteninformationen, insbesondere Größe und/oder Gewicht und/oder Geschlecht, abgeleitet werden können. Mit besonderem Vorteil kann die Registrierung auch die Anpassung des generisch vorgegebenen Patientenmodells auf den aktuellen Patienten umfassen.

Die Registrierung kann konkret unter Verwendung von Sensordaten wenigstens eines auf den Patienten gerichteten Sensors und/oder von mit der Bildaufnahmeeinrichtung aufgenommenen Bilddaten des Patienten und/oder aufgrund von aus einem Informationssystem abgerufenen Patientendaten und/oder aufgrund einer Ausgestaltung einer Patientenliege der Bildaufnahmeeinrichtung erhaltenen Patientenlagedaten erfolgen. Als Sensor kann hierbei in konkreter Ausgestaltung eine Kamera, insbesondere eine 3D-Kamera und/oder eine Infrarot-Kamera und/oder Terahertz-Kamera, und/oder ein Ultraschallsensor verwendet werden, wobei auch andere Ausgestaltungen möglich sind. Konkret können mithin eine Vielzahl von Methoden eingesetzt werden, um eine Registrierung des Patienten mit einem Koordinatensystem der Bildaufnahmeeinrichtung herbeizuführen, die sich insbesondere konkret in einer Registrierung des auf den aktuellen Patienten angepassten Patientenmodells mit diesem Koordinatensystem äußert.

So ist es zunächst denkbar, eine kamerabasierte, optische Registrierung vorzunehmen, wobei die Kamera den auf der Patientenliege befindlichen Patienten abbildet und eine Korrelation des Patientenumrisses bezogen auf die Patientenliege erstellt, welche insbesondere auch die Lage des Patienten ("Head First", "Feet First", "Supine", "Prone", ...) beschreibt. Die Verwendung einer Infrarot-Kamera hat hier den Vorteil, steril eine mögliche Abdeckung des Patienten durchschauen zu können. Auch eine ultraschallbasierte Erkennung der Lage des Patienten gegenüber der Patientenliege ist denkbar. Im Rahmen der Registrierung können hierbei auch aus einem Informationssystem, beispielsweise einem Krankenhausinformationssystem (HIS) und/oder einem Radiologieinformationssystem (RIS), erhaltene Patientendaten und/oder an der Bildaufnahmeeinrichtung eingegebene Patientendaten herangezogen werden, die unmittelbar beschreiben können, wie beispielsweise die Lage des Patienten auf der Patientenliege ist oder aber auch den Patienten genauer beschreiben können, beispielsweise durch Größe, Alter, Geschlecht, Gewicht und dergleichen, wobei derartige Patientendaten bevorzugt zur Anpassung eines generisch vorgegebenen Patientenmodells an den aktuellen Patienten genutzt werden können. Zusätzlich oder als Alternative ist auch eine bildbasierte Registrierung denkbar, beispielsweise die Verwendung eines ersten Vorabbildes und/oder der ersten Bilddaten, die von dem Patienten aufgenommen werden. Aus den Bilddaten können Informationen beispielsweise anhand von Datenbanken und/oder künstlicher Intelligenz extrahiert werden, wobei auch eine Organidentifikation denkbar ist. Schließlich kann im Rahmen der vorliegenden Erfindung auch eine Registrierung aufgrund einer Ausgestaltung der Patientenliege erfolgen, wobei in einer konkreten Ausführungsform vorgesehen sein kann, einen Patiententisch mit einer integrierten drucksensitiven Folie zu verwenden, die die wesentlichen Auflagepunkte des Patienten, insbesondere Kopf, Schultern, Gesäß, Waden, Füße und Arme erkennt, und zur Registrierung mit dem Patientenmodell korreliert.

Dabei sei nochmals hervorgehoben, dass die Anpassung des verwendeten (generisch vorgegebenen) Patientenmodells an den aktuellen Patienten bevorzugt aufgrund bei der Registrierung erhobener Registrierungsdaten erfolgt, so dass in dem Patientenmodell enthaltene Merkmale des Patienten in dem Koordinatensystem der Bildaufnahmeeinrichtung lokalisiert werden können. Mit anderen Worten stellt die Patientenregistrierung dann eine Beziehung zwischen den Koordinatensystemen des Patientenmodells (und somit des Patienten) und der Bildaufnahmeeinrichtung her, wobei durch das Patientenmodell beschriebene anatomische Merkmale somit bezüglich der Bildaufnahmeeinrichtung, insbesondere deren Aufnahmeanordnung, lokalisiert werden können, um im Ermittlungsalgorithmus entsprechende Schlussfolgerungen zu ziehen.

Im Rahmen der vorliegenden Erfindung kann vorgesehen sein, dass das Bildaufnahmeziel beschreibende Eingangsdaten aus einem Informationssystem abgerufen werden und/oder das Bildaufnahmeziel aus anderen Eingangsdaten abgeleitet wird, insbesondere aus einem innerhalb des Sichtfelds der Bildaufnahmeeinrichtung positionierten Anteil des Patienten. Das Bildaufnahmeziel (Zielapplikation) lässt sich dabei mit besonderem Vorteil aus Einträgen eines Krankenhausinformationssystems (HIS) und/oder Radiologieinformationssystems (RIS) ermitteln, nachdem entsprechende Informationen meist bei der Patientenregistrierung des Informationssystems erhalten werden. Allerdings ist es auch besonders vorteilhaft, entsprechende Schlussfolgerungen aus dem im Sichtfeld der Bildaufnahmeeinrichtung positionierten Anteil des Patienten zu ziehen, was aufgrund der Registrierung des Patientenmodells, wie ausführlich beschrieben, möglich ist. Der im Sichtfeld der Bildaufnahmeeinrichtung positionierte Anteil kann hierbei auch zur Plausibilisierung herangezogen bzw. analysiert werden.

In einer besonders vorteilhaften Ausgestaltung der vorliegenden Erfindung kann vorgesehen sein, dass die Eingangsdaten auch wenigstens ein für den Patienten bei dem Bildaufnahmevorgang verwendetes medizinisches Instrument beschreibende Instrumentendaten umfassen. Verwendete Instrumente ("Devices") können nicht nur einen Einfluss auf die Bildgebung der Bildgebungsart der Bildaufnahmeeinrichtung selbst haben, sondern insbesondere auch durch ihre Bildgebungseigenschaften bestimmte Anforderungen an die Bildaufnahme stellen, gerade dann, wenn es um die Abbildung des medizinischen Instruments selbst geht, beispielsweise bei der Nachverfolgung eines Katheters, bei der Überprüfung des Sitzes eines Implantates und dergleichen. Entsprechende, das medizinische Instrument beschreibende Eingangsdaten sind mithin nützliche Informationen bei der Ermittlung der Betriebsparameter durch den Ermittlungsalgorithmus.

Konkret können die in Instrumentendaten unter Verwendung einer einen Informationsträger auf dem Instrument und/oder einer Verpackung des Instruments auslesenden Erkennungseinrichtung und/oder von Instrumentendaten eines Informationssystems und/oder durch Auswertung von das Instrument zeigenden Bilddaten der Bildaufnahmeeinrichtung ermittelt werden und/oder wenigstens eine für die Bildgebungsart der Bildaufnahmeeinrichtung relevante Eigenschaft umfassen. Beispielsweise kann vorgesehen sein, die medizinischen Instrumente sozusagen an der Bildaufnahmeeinrichtung zu registrieren, indem mittels einer geeigneten Erkennungseinrichtung ein Informationsträger des medizinischen Instruments ausgelesen wird, beispielsweise ein Strichcode und/oder ein RFID-Chip. Zusätzlich oder alternativ kann auch eine Abfrage von Informationen eines Informationssystems und/oder ein bildbasierter Abgleich erfolgen, wobei beispielsweise das medizinische Instrument innerhalb von Bilddaten der Bildaufnahmeeinrichtung durch Segmentierungsalgorithmen detektiert werden kann, was insbesondere auch die Ermittlung von Eigenschaften des medizinischen Instruments ermöglicht. Entsprechend ist es auch besonders vorteilhaft, wenn zu den medizinischen Instrumenten Eigenschaften hinterlegt sind, insbesondere solche, die für die Bildgebungsart der Bildaufnahmeeinrichtung relevant sind, umfassend. Beispielsweise können Eigenschaften der medizinischen Instrumente wenigstens ein Material des medizinischen Instruments (beispielsweise Platin, Kunststoff, Eisen, Tantal, Iod, etc.), die geometrische Ausdehnung zumindest in einer Dimension (beispielsweise 1.2 French-Katheter, also ein Katheter mit einem Durchmesser von 0,4 mm, Stentdurchmesser, Durchmesser einer Stentstrebe, etc.) verwendet werden, um hierauf basierend Betriebsparameter der Bildaufnahmeeinrichtung anzupassen.

Soll in einem Beispiel ein kleiner Katheter verwendet werden, kann es zweckmäßig sein, die Betriebsparameter hinsichtlich einer besseren Auflösung anzupassen, zusätzlich zu einer gegebenenfalls bereits gegebenen Optimierung von Betriebsparametern hinsichtlich des Einsatzbereichs des Katheters, so dass beispielsweise unterschiedliche Bildaufnahmeparameter und/oder Bildauswertungsparameter herangezogen werden, wenn sich das medizinische Instrument im Leberbereich des Patienten oder im Herzbereich des Patienten befindet. Auch weitere Informationen über das Zielgebiet des medizinischen Instruments können relevant sein, beispielsweise Embolisation im Zielgebiet und dergleichen.

Zweckmäßigerweise sind im Rahmen der vorliegenden Erfindung die Positionen und/oder Ausrichtungen von bewegbaren Komponenten der Bildaufnahmeeinrichtung, insbesondere von bewegbaren Komponenten der Aufnahmeanordnung und/oder der Patientenliege, bekannt, mithin auch deren Positionen und/oder Orientierungen (Posen) im Koordinatensystem der Bildaufnahmeeinrichtung. Derartige, auch als Eingangsdaten verwendbare und/oder anderweitig in den Ermittlungsalgorithmus eingehende Komponenteninformationen können auf unterschiedliche Art und Weise gewonnen werden, so dass beispielsweise das Vorhandensein und/oder Positionen/Orientierungen von Komponenten der Bildaufnahmeeinrichtung aus der Stellung von diesen zugeordneten Aktoren und/oder durch den Komponenten zugeordnete Sensoren ermittelt werden. Im Gegensatz zum Stand der Technik können hier selbstverständlich auch weitere Sensoren hinzugefügt werden, insbesondere bei Komponenten, die manuell platziert und/oder eingestellt werden. Dabei können beispielsweise Hilfsmittel, insbesondere Filter und/oder Kollimatoren und/oder Raster, ähnlich wie medizinische Instrumente behandelt werden, insbesondere auch entsprechend in (ersten) Bilddaten detektiert und/oder mittels Informationsträgern bei Verwendung registriert werden.

Zweckmäßigerweise werden die Eingangsdaten zyklisch und/oder bei Veränderungen aktualisiert, wobei eine Anpassung wenigstens eines der Betriebsparameter aufgrund der geänderten Eingangsdaten erfolgt. Das bedeutet, die beschriebenen Eingangsdaten können, falls nötig, bildsynchron oder zumindest von Zeit zu Zeit aktualisiert werden. Beispielsweise kann sich während einer Bildsequenz in einem Aufnahmevorgang die Angulation eines C-Bogens und/oder allgemein die Position einer Patientenliege verändern, so dass sich die Aufnahmebedingungen und/oder der Untersuchungsbereich verändern. Ein konkretes Beispiel hierfür umfasst das Einführen eines Katheters bzw. Führungsdrahtes beim Zugang (Lende oder radial), wobei der Katheter bzw. Führungsdraht über die Aorta (oder Radialis) zum Aortenbogen geschoben wird, während die Patientenliege mitbewegt wird, so dass die Spitze des medizinischen Instruments immer in den aufgenommenen Bilddatensatz zu sehen ist und dabei verschiedene Organe/Untersuchungsbereiche überstreicht. Hier ist es mit besonderem Vorteil möglich, immer die geeigneten Betriebsparameter dynamisch mittels des Ermittlungsalgorithmus zu bestimmen und somit verbesserte Bildqualität für alle Positionen des medizinischen Instruments bereitzustellen, indem die Eingangsdaten und die daraus folgenden Betriebsparameter immer aktuell gehalten werden.

In einer zweckmäßigen Weiterbildung kann durch den Ermittlungsalgorithmus wenigstens eine für die verwendete Bildgebungsart der Bildaufnahmeeinrichtung relevante Eigenschaft des Aufnahmebereichs des Patienten ermittelt und zur Anpassung wenigstens eines Betriebsparameters, insbesondere eines Bildauswertungsparameters, verwendet werden.

Dies sei an einem konkreten Beispiel für eine Röntgeneinrichtung als Bildaufnahmeeinrichtung näher erläutert. Beispielsweise kann die Ermittlung der Betriebsparameter einer Optimierung des Röntgenspektrums für das Bildaufnahmeziel umfassen. Dabei basiert die heutige Dosisregelung und spektrale Anpassung auf einem generell verwendeten Mischmodell der wesentlichen Patientenmaterialeigenschaften, wobei beispielsweise eine bestimmte Zusammensetzungsannahme getroffen wird. Mithilfe der vorliegenden Eingangsdaten, insbesondere der Ermittlung des Anteils des Patienten im Sichtfeld der Bildaufnahmeeinrichtung, wobei zusätzlich aus dem Patientenmodell die dem Anteil entsprechende Anatomie bekannt ist, kann nun wesentlich dedizierter eine realistischere Zusammensetzung des Patienten im im Sichtbereich liegenden Anteil angesetzt werden, so dass die dadurch erwarteten Primär- und Streustrahlenanteile durch geeignete Röntgenspektrenwahl (Röhrenspannung, Vorfilterung, etc.) optimiert werden können.

Dabei sei angemerkt, dass die Eingangsdaten, insbesondere das Patientenmodell, auch gegebenenfalls vorhandene Implantate des Patienten beschreiben können, die ebenso bei der Auswahl bzw. Ermittlung geeigneter Betriebsparameter berücksichtigt werden können. So können beispielsweise die Informationen über bekannte Implantate (Knie, Hüfte, etc.) genauso zur Optimierung des Röntgenspektrums und der Röntgendosis herangezogen werden, wie im Übrigen auch Informationen über Einblendungen von Kollimatoren und/oder verwendete Filter.

Insbesondere können als Eingangsdaten oder Betriebsparameter mithin allgemein zu verwendende Filter und/oder Kollimatoren und/oder deren Position und/oder deren Einstellung ermittelt werden. Dabei sei darauf hingewiesen, dass Informationen zu Filtern/Kollimatoren wie auch zu Implantaten auch durch Bildanalyse beispielsweise von Vorabbildern oder ersten aufgenommenen Bilddaten des Patienten ermittelt werden können.

Auch weitere physikalische Betriebsparameter, mithin Bildaufnahmeparameter, können im Übrigen aufgrund des Wissens über den Anteil des Patienten im Sichtfeld zur Optimierung angepasst werden, insbesondere hinsichtlich des OOI (Organ of Interest). Beispielsweise kann aufgrund der Eingangsdaten und der Registrierung mit einer hohen Wahrscheinlichkeit als Bildaufnahmeziel eine Angiographie der rechten Koronararterie oder der linken Koronararterie vorausgesagt werden und automatisch eine optimierte Wahl der Pulslänge als Betriebsparameter erfolgen. Denn im Beispiel der Koronararterien ist bekannt, dass die linken und die rechten Koronararterien deutlich unterschiedliche Bewegungsgeschwindigkeiten zeigen. Hierbei bewegt sich die rechte Koronararterie deutlich schneller als die linke Koronararterie. Ist die Information, welche Koronararterie untersucht werden soll, bekannt (folgt aus Registrierung, Patientenmodell, Sichtfeld der Bildaufnahmeeinrichtung) und gegebenenfalls dem Bildaufnahmeziel (Zielapplikation - Kardangiographie), kann eine entsprechende Anpassung der Pulslänge der Röntgenpulse und/oder der Bildfrequenz an den Betriebsparameter erfolgen, um die zeitliche Auflösung zu optimieren und Bewegungsartefakte zu vermeiden bzw. zu vermindern.

Ein anderes nützliches Anwendungsgebiet für das beschriebene Vorgehen sind mehrere Einzelbildaufnahmen, die zeitlich aufeinander folgen, umfassende Bildaufnahmevorgänge. Für die einzelnen Bildaufnahmezeitpunkte können (aufgrund der Registrierung und des Patientenmodells) die Aufnahmegeometrie bezüglich des Patienten beschreibende physikalische Betriebsparameter ermittelt werden, beispielsweise Systempositionen umfassend die Position einer Patientenliege und/oder einer Aufnahmeanordnung (jeweils ggf. inklusive Orientierung). Insbesondere nach einer Freigabe durch einen Bediener werden die Systempositionen nacheinander angefahren und die Bildaufnahmen erfolgen. Derartiges ist beispielsweise in der Koronardiagnostik bei Röntgeneinrichtungen, insbesondere mit einem C-Bogen, üblich. Die Systempositionen können dann die Position einer Patientenliege und die Angulation eines C-Bogens umfassen. Wie andere Betriebsparameter durch einen Algorithmus der künstlichen Intelligenz bestimmt werden können, so gilt dies auch für derartige Abfolgen von Systempositionen (oder im Allgemeinen Aufnahmegeometrien). Trainingsdaten können beispielsweise bei gleichartigen Untersuchungen anderer Patienten gewonnen werden.

Die optimierende Bestimmung von Betriebsparametern beschränkt sich jedoch nicht auf physikalische Betriebsparameter (Bildaufnahmeparameter), sondern kann sich auch auf Bildauswertungsparameter (bildverarbeitungstechnische Parameter) beziehen. Beispielsweise kann abhängig von den durch den Ermittlungsalgorithmus gewählten physikalischen Betriebsparametern und/oder von Informationen über verwendete medizinische Instrumente und/oder Implantatinformationen die Bildverarbeitung bildsynchron optimiert werden, beispielsweise was die Stärke der temporalen und bildbasierten Rauschunterdrückung, das Kontrast- und Dynamikmanagement, die ortsfrequenzabhängige Bildschärfe und dergleichen angeht.

In einer weiteren, besonders vorteilhaften Ausgestaltung der vorliegenden Erfindung kann vorgesehen sein, dass die Betriebsparameter die automatische Ausgabe wenigstens eines Workflow-Hinweises beschreiben, insbesondere bezüglich eines zu verwendenden Hilfsmittels bei der Bildaufnahme und/oder zur Minimierung einer negativen Auswirkung auf den Patienten. Das bedeutet, Betriebsparameter können sich auch auf die Interaktion mit einem Benutzer beziehen, indem dieser beispielsweise bei der Durchführung eines dem Bildaufnahmevorgang zugeordneten Workflows unterstützt wird. Beispielsweise kann er Workflow-Hinweise über entsprechende Ausgabemittel erhalten, insbesondere die Art und/oder Positionierung eines zu verwendenden Hilfsmittels bei der Bildaufnahme und/oder Hinweise zur optimalen Behandlung des Patienten während des Bildaufnahmevorgangs.

Beispielsweise kann aus den zu untersuchenden Patienten beschreibenden Eingangsdaten erkannt werden, ob es sich um einen pädiatrischen Patienten, beispielsweise ein Kleinkind, oder einen Erwachsenen handelt. Hieraus kann beispielsweise ein Workflow-Hinweis folgen, dass Streustrahlenraster zu verwenden oder bei pädiatrischen Untersuchungen aus dem Strahlengang zu entfernen, wenn die Bildaufnahmeeinrichtung als eine Röntgeneinrichtung ausgebildet ist. Ebenso ist es bei Röntgeneinrichtungen äußerst nützlich, bildaufnahmeeinrichtungsseitig automatisiert Workflow-Hinweise zu erzeugen, die sich auf eine Optimierung der lokalen Dosisbelastung, insbesondere der Hautdosis, beziehen. Dabei kann beispielsweise in Workflow-Hinweisen ein Wechsel von Aufnahmegeometrien, beispielsweise zu Komplementärangulationen, vorgeschlagen werden, um bei länger andauernden Bildaufnahmevorgängen die lokal applizierte Hautdosis für den Patienten zu minimieren. Bei anderen Bildaufnahmeeinrichtungen, beispielsweise einer Magnetresonanzeinrichtung als Bildaufnahmeeinrichtung, kann beispielsweise eine diesbezügliche Workflow-Hinweisgabe sich auf die lokale und/oder globale SAR beziehen.

Neben dem Verfahren betrifft die Erfindung auch eine Bildaufnahmeeinrichtung, insbesondere eine Röntgeneinrichtung, aufweisend eine Aufnahmeanordnung und eine zur Durchführung eines erfindungsgemäßen Verfahrens ausgebildete Steuereinrichtung. Sämtliche Ausführungen bezüglich des erfindungsgemäßen Verfahrens lassen sich analog auf die erfindungsgemäße Bildaufnahmeeinrichtung übertragen, mit welcher mithin ebenso die bereits genannten Vorteile erhalten werden können. Die Steuereinrichtung kann wenigstens einen Prozessor und/oder wenigstens ein Speichermittel umfassen. Zur Durchführung des erfindungsgemäßen Verfahrens kann die Steuereinrichtung insbesondere eine Ermittlungseinheit aufweisen, die den Ermittlungsalgorithmus implementiert. Ferner kann auch eine Registrierungseinheit zur Registrierung des Patienten, insbesondere mittels des Patientenmodells, mit einem Koordinatensystem der Bildaufnahmeeinrichtung vorhanden sein. Die Steuerung der Bildaufnahmeeinrichtung durch Umsetzung der Betriebsparameter kann mittels einer entsprechenden Steuereinheit erfolgen.

Ein erfindungsgemäßes Computerprogramm ist beispielsweise direkt in einen Speicher einer Steuereinrichtung einer Bildaufnahmeeinrichtung ladbar und weist Programmmittel auf, um die Schritte eines erfindungsgemäßen Verfahrens auszuführen, wenn das Computerprogramm in der Steuereinrichtung der Bildaufnahmeeinrichtung ausgeführt wird. Das Computerprogramm kann auf einem erfindungsgemäßen elektronisch lesbaren Datenträger gespeichert sein, welcher mithin darauf gespeicherte elektronisch lesbare Steuerinformationen umfasst, welche zumindest ein genanntes Computerprogramm umfassen und derart ausgestaltet sind, dass sie bei Verwendung des Datenträgers in einer Steuereinrichtung einer Bildaufnahmeeinrichtung ein erfindungsgemäßes Verfahren durchführen. Bei dem Datenträger kann es sich um einen nichttransienten Datenträger, insbesondere eine CD-ROM, handeln.

Weitere Vorteile und Einzelheiten der vorliegenden Erfindung ergeben sich aus den im Folgenden beschriebenen Ausführungsbeispielen sowie anhand der Zeichnungen. Dabei zeigen:
- Fig. 1: einen schematischen Ablaufplan des erfindungsgemäßen Verfahrens, und
- Fig. 2: eine erfindungsgemäße Bildaufnahmeeinrichtung in Form einer Röntgeneinrichtung.

Im Folgenden werden Ausführungsbeispiele der vorliegenden Erfindung mit Bezug auf eine Röntgeneinrichtung als Bildaufnahmeeinrichtung diskutiert, wobei eine Anwendung selbstverständlich auch auf andere Bildaufnahmeeinrichtungen höherer Komplexität, beispielsweise Magnetresonanzeinrichtungen, denkbar ist.

Die grundlegende Idee dabei ist es, auf Basis eines Teils oder aller vorliegenden Eingangsinformationen wie auf den aktuellen Patienten angepasstes Patientenmodell, Registrierung des Patientenmodells und somit des Patienten mit einem Koordinatensystem der Bildaufnahmeeinrichtung, Kenntnis des Bildaufnahmeziels, Informationen zum aktuellen Untersuchungsbereich (Organ of Interests - OOI), Informationen zu Implantaten, Direktstrahlung, Lage von Filtern/Kollimatoren, Einbeziehung erwarteter Bewegungen im Untersuchungsbereich, Wissen über verwendete medizinische Instrumente bzw. das für die Bildgebung kritischsten medizinischen Instruments, möglichst optimale physikalische und bildverarbeitungstechnische Betriebsparameter seitens einer Steuereinrichtung der Bildaufnahmeeinrichtung vollständig automatisch auszuwählen. Hierzu wird ein befähigter Ermittlungsalgorithmus verwendet, der bevorzugt die komplette bildrelevante Parametrierung der Bildaufnahmeeinrichtung vornimmt, so dass keinerlei Benutzeraktion, beispielsweise die Anwahl eines Messprotokolls/Organprogramms, notwendig ist.

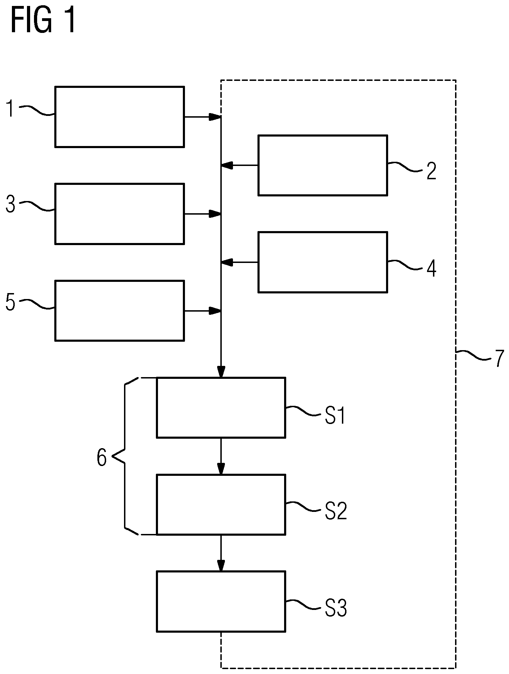

Diese Grundlage des erfindungsgemäßen Vorgehens wird mithilfe des Ablaufplans der Fig. 1 näher erläutert. Die Eingangsinformationen für den Ermittlungsalgorithmus, der auch wenigstens einen Algorithmus der künstlichen Intelligenz umfassen kann, umfassen vorliegend neben der bereits erwähnten Registrierung 1 des Patienten verschiedene Eingangsdaten 2 bis 5, wobei zunächst das Patientenmodell (Eingangsdaten 2) näher erläutert sei.

Im vorliegend beschriebenen Ausführungsbeispiel wird ein grundsätzlich generisches Patientenmodell verwendet, das aufgrund der Registrierung 1 auf den aktuellen Patienten angepasst und mit dem Koordinatensystem der Bildaufnahmeeinrichtung registriert wird. Dabei kann als Patientenmodell ein computertomographieartiges Patientenmodell, das alle wichtigen Organe und das Blutgefäßsystem abbildet, und/oder ein vektorbasiertes einfaches Patientenmodell, das die Umrisse eines generischen Patienten und die Lage aller wichtigen Organe auf die Umrisse bezogen abbildet, verwendet werden. Durch die im Rahmen der Registrierung erhaltenen Registrierungsdaten wird nicht nur die Position und die Lage des Patienten beschrieben, sondern auch dessen weitere relevante Eigenschaften, insbesondere seine Ausdehnungen/Umrisse, so dass eine Anpassung des Patientenmodells auf den aktuellen Patienten und aufgrund der Registrierung auch eine passende Positionierung des Patientenmodells zu dem Koordinatensystem der Bildaufnahmeeinrichtung problemlos algorithmisch auf die Steuereinrichtung der Bildaufnahmeeinrichtung möglich ist. Die so beschriebene Kombination von Patientenmodell und Registrierung stellt eine der wesentlichen Grundlagen des hier beschriebenen Vorgehens dar.

Zur Herstellung der Registrierung 1 können verschiedenste Vorgehensweisen, insbesondere auch kombiniert, eingesetzt werden, beispielsweise ein kamerabasiertes optisches System, das den Patienten auf der Patientenliege der Bildaufnahmeeinrichtung abbildet und eine Korrelation des Patientenumrisses bezogen auf die Patientenliege erstellt, ein infrarotbasiertes System, welches ähnlich der im sichtbaren Bereich messenden Kamera arbeitet, jedoch Abdeckungen ähnlich wie eine Terahertz-Kamera durchmessen kann, ein ultraschallbasiertes Erkennungssystem mit entsprechenden Ultraschallsensoren, die Verwendung einer in die Patientenliege integrierten drucksensitiven Folie, die die wesentlichen Auflagepunkte vermessen kann und mit dem Patientenmodell korrelieren kann, sowie Zusatzinformationen über den Patienten, beispielsweise Eingaben bezüglich der Lage des Patienten auf der Patientenliege sowie patientenspezifische Patientendaten, insbesondere Größe, Alter, Geschlecht, Gewicht und dergleichen. Patientendaten dieser Art können beispielsweise aus Informationssystemen, insbesondere aus einem Krankenhausinformationssystem (HIS) und/oder einem Radiologieinformationssystem (RIS) abgerufen werden.

Weitere Eingangsdaten 3 beschreiben den Systemzustand der Bildaufnahmeeinrichtung, insbesondere die durch die Aufnahmeanordnung definierte aktuelle Aufnahmegeometrie. Bei der Röntgeneinrichtung kann hierbei beispielsweise die Positionierung des Röntgenstrahlers und des Röntgendetektors als Aufnahmeanordnung, die beispielsweise von einem C-Bogen getragen werden kann, zur Patientenliege beschrieben sein, was eine äußerst wichtige Information im Rahmen der hier beschriebenen Ausführungsbeispiele darstellt, da in Zusammenschau mit dem im Koordinatensystem der Bildaufnahmeeinrichtung vorliegenden Patientenmodell (Eingangsdaten 2, Registrierung 1), welches relevante Organe/anatomische Strukturen des Patienten beschreibt, unmittelbar festgestellt werden kann, welcher Anteil des Patienten sich im Blickfeld der Bildaufnahmeeinrichtung befindet, was einen deutlichen Hinweis auf das Bildaufnahmeziel bzw. das Organ of Interest (OOI) darstellt. Weitere Eingangsdaten 5, die den Bildaufnahmevorgang bzw. konkret das Bildaufnahmeziel betreffen, können beispielsweise wiederum aus einem der bereits erwähnten Informationssysteme abgerufen werden. Durch eine entsprechende Kombination des Wissens über den Anteil des Patienten im Sichtfeld (Registrierung 1, Eingangsdaten 2, 3) mit den weiteren Eingangsdaten 5, die das Bildaufnahmeziel betreffen, entsteht ein besonders genaues Wissen über die voraussichtlich benötigten Betriebsparameter bzw. deren Werte. Geben beispielsweise die Eingangsdaten 5 an, dass eine Kardangiographie durchgeführt werden soll, und spricht der sich im Sichtfeld der Bildaufnahmeeinrichtung befindliche Anteil des Patienten dafür, dass die rechte Koronararterie aufgenommen werden soll, kann das Hintergrundwissen, dass sich die rechte Koronararterie schneller bewegt als die linke Koronararterie genutzt werden, um beispielsweise entsprechend kurze Pulse oder eine entsprechende Bildfrequenz zu wählen, um Bewegungsartefakte zu minimieren und die zeitliche Auflösung zu optimieren.

Bezüglich der Eingangsdaten 3 (Betriebszustand der Bildaufnahmeeinrichtung) sei noch angemerkt, dass entsprechende Informationen häufig ohnehin als Rückmeldung angesteuerter Aktoren und/oder durch entsprechende Sensoriken in der Steuereinrichtung der Bildaufnahmeeinrichtung vorliegen bzw. leicht ergänzt werden können. Die Eingangsdaten 3 können im Übrigen auch Informationen zu verwendeten Hilfsmitteln/optionalen und/oder einstellbaren Komponenten umfassen, insbesondere hinsichtlich von Kollimatoren und/oder Filtern. Derartige Eingangsdaten 3 lassen sich im Übrigen auch aus Bilddaten der Bildaufnahmeeinrichtung selber ableiten.

Aus Bilddaten abgeleitet oder aus einem Informationssystem abgerufen werden können im Übrigen auch Informationen zu Implantaten des Patienten, die zu den Eingangsdaten 2 gehörig ermittelt werden können. Gerade bei der Röntgenbildgebung als Bildgebungsart können Implantate aufgrund ihrer Schwächungseigenschaften Einfluss nehmen, so dass Betriebsparameter auf das Vorhandensein derselben abgestellt werden können.

Eine weitere äußerst nützliche Eingangsinformation stellen die Eingangsdaten 4 dar, die bei dem Bildaufnahmevorgang insbesondere an und/oder im Patienten genutzte medizinische Instrumente ("Devices") betrifft. Derartige medizinische Instrumente können durchaus auch in Zusammenhang mit dem Bildaufnahmeziel stehen und/oder Einfluss auf die Bildaufnahme nehmen, weshalb das Wissen über sie äußerst nützlich sein kann. Informationen zu verwendeten Devices können durch das Auslesen von Informationsträgern, beispielsweise Strichcodes, mittels einer entsprechenden Erkennungseinrichtung ermittelt werden, wobei optional ein bildbasierter Abgleich der Instrumenteneigenschaften, gegebenenfalls mithilfe von Segmentationsalgorithmen, erfolgen kann. Die Eingangsdaten 4 umfassen auch zu den medizinischen Instrumenten hinterlegte Eigenschaften wie beispielsweise das Material, die geometrische Ausdehnung in wenigstens einer Dimension und dergleichen, die bei der Ermittlung der Betriebsparameter ebenso genutzt werden können.

Die letztendliche Durchführung des Ermittlungsalgorithmus 6 erfolgt in zwei Schritten S1 und S2. Im Schritt S1 werden zunächst physikalische Betriebsparameter, die auch als Bildaufnahmeparameter bezeichnet werden können und den physikalischen Bildaufnahmevorgang betreffen, ermittelt. Auch in deren Abhängigkeit werden in einem Schritt S2 dann bildverarbeitungstechnische Betriebsparameter (Bildauswertungsparameter) ermittelt. Wie bereits erwähnt, kann der Ermittlungsalgorithmus 6 wenigstens einen Algorithmus der künstlichen Intelligenz umfassen, der beispielsweise durch Trainingsdaten trainiert wird, die bei der Verwendung von Bildaufnahmeeinrichtungen, die messprotokollbasiert arbeiten, durch Protokollierung ermittelt werden können. Künstliche Intelligenz bietet sich insbesondere bei komplexeren Teilsachverhalten an, während sich viele der beschriebenen Sachverhalte, wie durch die Beschreibung angedeutet, auch ohne die Verwendung künstlicher Intelligenz bereits erschließen lassen.

In konkreten Ausführungsbeispielen ist es beispielsweise möglich, die Röntgendosis und das Röntgenspektrum beschreibende Betriebsparameter als Bildaufnahmeparameter zu ermitteln, indem anhand des bekannten Anteils des Patienten im Sichtfeld (und gegebenenfalls des Wissens über Implantate) eine Zusammensetzungsinformation des durchstrahlten Bereichs ermittelt wird, anhand derer die entsprechenden Betriebsparameter gewählt werden können, wobei in diesem Kontext auch künstliche Intelligenz-Ansätze herangezogen werden können. Eine Optimierung kann beispielsweise hinsichtlich der erwarteten Primär- und Streustrahlenanteile erfolgen. Das Wissen über Implantate kann ferner auch Einblendungen von Kollimatoren und/oder die Verwendung von Filtern zur Optimierung des Röntgenspektrums beeinflussen. Was die Pulslänge und/oder die Bildaufnahmefrequenz als Betriebsparameter angeht, können beispielsweise Informationen zur Bewegung des interessierenden Organs (OOI) ermittelt und entsprechend ausgewertet werden. Abhängig von den gewählten physikalischen Betriebsparametern sowie den Eingangsdaten 2 bis 5 werden dann im Schritt S2 Bildauswertungsparameter als Betriebsparameter ermittelt, beispielsweise die Stärke der temporalen und bildbasierten Rauschunterdrückung, das Kontrast- und das Dynamikmanagement beschreibende Parameter, die ortsfrequenzabhängige Bildschärfe und dergleichen.

Es sei an dieser Stelle noch angemerkt, dass als weitere Klasse von Betriebsparametern, beispielsweise in einem weiteren, hier der Übersichtlichkeit halber nicht näher dargestellten Schritt des Ermittlungsalgorithmus 6, auch auf auszugebende Workflow-Hinweise bezogene Betriebsparameter ermittelt werden können. Durch solche Workflow-Hinweise, die automatisch erzeugt werden, kann der Benutzer auf zu verwendende oder auch nicht verwendende Komponenten/Hilfsmittel, beispielsweise ein Streustrahlenraster, hingewiesen werden, und/oder es kann durch entsprechende Workflow-Hinweise an den Benutzer eine Hautdosis-Minimierung oder dergleichen erreicht werden.

In einem Schritt S3 werden die ermittelten Betriebsparameter dann zur Steuerung der Bildaufnahmeeinrichtung, insbesondere also zur Bildakquisition, Bildverarbeitung, Bilddarstellung und Bildspeicherung, verwendet.

Wie der Pfeil 7 andeutet, können die beschriebenen Eingangsdaten bildsynchron aktualisiert werden, beispielsweise, falls sich während einer Bildsequenz die Aufnahmegeometrie und/oder die Position der Patientenliege ändert. Grund hierfür kann beispielsweise die Bewegung eines medizinischen Instruments, das nachverfolgt werden soll, innerhalb des Patienten sein. So können immer auf die aktuellen Umstände möglichst optimal gewählte Betriebsparameter mittels des Ermittlungsalgorithmus 6, der dann entsprechend auch bildsynchron durchgeführt wird, ermittelt werden.

Es sei angemerkt, dass die Betriebsparameter auch automatisch anzufahrende Positionen der Patientenliege (und somit (aufgrund der Registrierung bekannt) auch des Patienten) und/oder der Aufnahmeanordnung, zusammengefasst Systempositionen, sowie deren Anzahl für aufeinanderfolgende Bildaufnahmen mit der Bildaufnahmeeinrichtung umfassen können. Beispielsweise können die Eingangsdaten 2 bis 5 beschreiben, dass eine Koronaruntersuchung stattfinden soll, insbesondere mit speziellen Anforderungen an die Bildgebung, ferner aber auch aufgrund des Patientenmodells die Position, Ausrichtung und Größe des Herzens beschreiben. Hieraus können für mehrere Bildaufnahmevorgänge umfassende Untersuchungen geeignete Aufnahmegeometrien bestimmt werden und als Betriebsparameter beispielsweise Patientenliegenpositionen und Aufnahmeanordnungspositionen, also bestimmte Systempositionen, beschreiben, die automatisch angefahren werden können.

Die Systempositionen können dabei beispielsweise im klinischen Alltag aus gleichartigen Untersuchungen beschreibenden Trainingsdaten maschinell gelernt werden. Hier, wie auch in anderen konkreten Anwendungsfällen, ist es möglich, beispielsweise klinikspezifisch und/oder benutzerspezifisch zu lernen und somit auch die entsprechend lokal und/oder individuell bevorzugten Betriebsparameter einzusetzen.

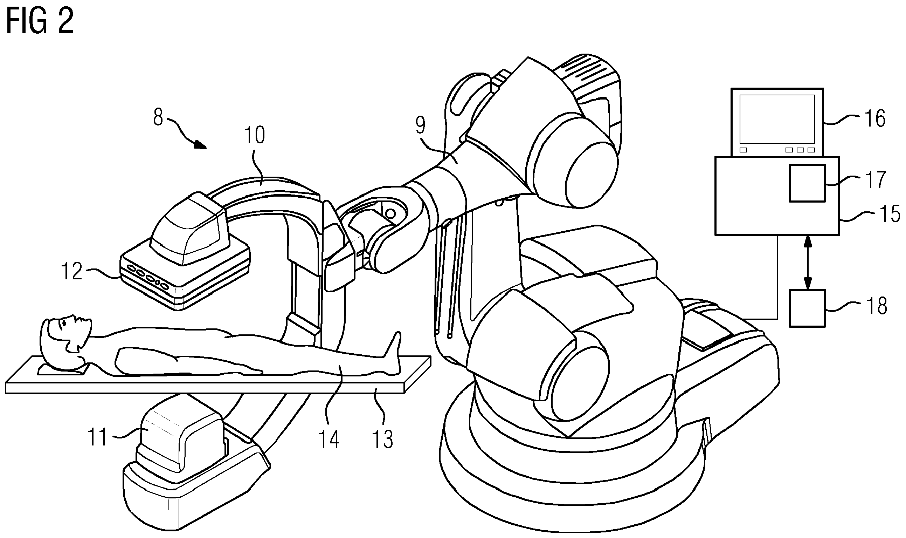

Fig. 2 zeigt schließlich eine Darstellung einer als interventionelle Röntgeneinrichtung ausgebildeten Bildaufnahmeeinrichtung 8, die vorliegend einen an einem beweglichen Roboterarm 9 gelagerten C-Bogen 10 umfasst, an dem als Aufnahmeanordnung sich gegenüberliegend ein Röntgenstrahler 11 und ein Röntgendetektor 12 angeordnet sind. Damit können verschiedene Aufnahmegeometrien bezüglich einer Patientenliege 13, die ebenfalls bewegbar ausgestaltet sein kann, realisiert werden, auf der der zu untersuchende und/oder zu behandelnde Patient 14 gelagert werden kann. Der Betrieb der Bildaufnahmeeinrichtung 8 wird dabei durch eine Steuereinrichtung 15 gesteuert, die auch zur Durchführung des erfindungsgemäßen Verfahrens ausgebildet ist.

Der Steuereinrichtung 15 kann ein Ausgabemittel 16 zur Ausgabe von Workflow-Hinweisen, beispielsweise ein Bildschirm und/oder ein akustisches Ausgabemittel, zugeordnet sein. Zur Ausführung des Ermittlungsalgorithmus 6 kann die Steuereinrichtung 15 insbesondere neben anderen Funktionseinheiten eine entsprechende Ermittlungseinheit 17 aufweisen. Die Steuereinrichtung 15 kann ferner zur Kommunikation mit externen Informationssystemen 18 ausgebildet sein.

Obwohl die Erfindung im Detail durch das bevorzugte Ausführungsbeispiel näher illustriert und beschrieben wurde, so ist die Erfindung nicht durch die offenbarten Beispiele eingeschränkt und andere Variationen können vom Fachmann hieraus abgeleitet werden, ohne den Schutzumfang der Erfindung zu verlassen.

## Patentansprüche

1. Verfahren zum Betrieb einer medizinischen Bildaufnahmeeinrichtung (8) im Rahmen eines einem Bildaufnahmeziel dienenden Bildaufnahmevorgangs an einem Patienten (14), wobei die Steuerung der Bildaufnahmeeinrichtung (8) auf Grundlage von durch eine Steuereinrichtung (15) der Bildaufnahmeeinrichtung (8) umzusetzenden Betriebsparametern erfolgt, **dadurch gekennzeichnet, dass** die Betriebsparameter mittels eines Ermittlungsalgorithmus (6) vollständig automatisch aus wenigstens den Patienten (14) in Form eines Patientenmodells und/oder das Bildaufnahmeziel beschreibenden Eingangsdaten (2, 3, 4, 5) und einer Registrierung (1) des Patienten (14) mit einem Koordinatensystem der Bildaufnahmeeinrichtung (8) ermittelt und zur Steuerung der Bildaufnahmeeinrichtung (8) verwendet werden.

2. Verfahren nach Anspruch 1, **dadurch gekennzeichnet, dass** der Ermittlungsalgorithmus (6) wenigstens einen Algorithmus der künstlichen Intelligenz umfasst, der durch maschinelles Lernen trainiert wurde.

3. Verfahren nach Anspruch 2, **dadurch gekennzeichnet, dass** Trainingsdaten für den Algorithmus der künstlichen Intelligenz durch Protokollierung einer insbesondere wenigstens eine Einzelparameteranpassung umfassenden Tätigkeit eines Benutzers bei der Verwendung von vordefinierten Messprotokollen mit vordefinierten Betriebsparametern ermittelt werden.

4. Verfahren nach einem der vorangehenden Ansprüche, **dadurch gekennzeichnet, dass** die Betriebsparameter neben Bildaufnahmeparametern auch wenigstens einen Bildauswertungsparameter umfassen.

5. Verfahren nach einem der vorangehenden Ansprüche, **dadurch gekennzeichnet, dass** als den Patienten (14) beschreibende Patientendaten wenigstens das auf den aktuellen Patienten (14) angepasste Patientenmodell verwendet wird, welches lokal aufgelöst die Zusammensetzung des Patienten (14) beschreibt.

6. Verfahren nach einem der vorangehenden Ansprüche, **dadurch gekennzeichnet, dass** die Registrierung (1) unter Verwendung von Sensordaten wenigstens eines auf den Patienten (14) gerichteten Sensors und/oder von mit der Bildaufnahmeeinrichtung (8) aufgenommenen Bilddaten des Patienten (14) und/oder aufgrund von aus einem Informationssystem (18) abgerufenen Patientendaten und/oder aufgrund einer Ausgestaltung einer Patientenliege (13) der Bildaufnahmeeinrichtung (8) erhaltenen Patientenlagedaten erfolgt.

7. Verfahren nach Anspruch 6, **dadurch gekennzeichnet, dass** aufgrund bei der Registrierung (1) erhobener Registrierungsdaten das verwendete Patientenmodell an den aktuellen Patienten (14) angepasst wird, so dass in dem Patientenmodell enthaltene Merkmale in dem Koordinatensystem der Bildaufnahmeeinrichtung (8) lokalisiert werden können.

8. Verfahren nach einem der vorangehenden Ansprüche, **dadurch gekennzeichnet, dass** das Bildaufnahmeziel beschreibende Eingangsdaten (2, 3, 4, 5) aus einem Informationssystem (18) abgerufen werden und/oder aus anderen Eingangsdaten (2, 3, 4, 5) abgeleitet werden, insbesondere aus einem innerhalb des Sichtfelds der Bildaufnahmeeinrichtung (8) positionierten Anteil des Patienten (14).

9. Verfahren nach einem der vorangehenden Ansprüche, **dadurch gekennzeichnet, dass** die Eingangsdaten (2, 3, 4, 5) auch wenigstens ein für den Patienten (14) bei dem Bildaufnahmevorgang verwendetes medizinisches Instrument beschreibende Instrumentendaten umfassen.

10. Verfahren nach Anspruch 9, **dadurch gekennzeichnet, dass** die Instrumentendaten unter Verwendung einer einen Informationsträger auf dem Instrument und/oder einer Verpackung des Instruments auslesenden Erkennungseinrichtung und/oder von Instrumentendaten eines Informationssystems (18) und/oder durch Auswertung von das Instrument zeigenden Bilddaten der Bildaufnahmeeinrichtung (8) ermittelt werden und/oder wenigstens eine für die Bildgebungsart der Bildaufnahmeeinrichtung (8) relevante Eigenschaft umfassen.

11. Verfahren nach einem der vorangehenden Ansprüche, **dadurch gekennzeichnet, dass** das Vorhandensein und/oder Positionen von Komponenten der Bildaufnahmeeinrichtung (8) aus der Stellung von diesen zugeordneten Aktoren und/oder durch den Komponenten zugeordnete Sensoren ermittelt werden.

12. Verfahren nach einem der vorangehenden Ansprüche, **dadurch gekennzeichnet, dass** die Eingangsdaten (2, 3, 4, 5) zyklisch und/oder bei Veränderungen aktualisiert werden, wobei eine Anpassung wenigstens eines der Betriebsparameter aufgrund der geänderten Eingangsdaten (2, 3, 4, 5) erfolgt.

13. Verfahren nach einem der vorangehenden Ansprüche, **dadurch gekennzeichnet, dass** durch den Ermittlungsalgorithmus (6) wenigstens eine für die verwendete Bildgebungsart der Bildaufnahmeeinrichtung (8) relevante Eigenschaft des Aufnahmebereichs des Patienten (14) ermittelt und zur Anpassung wenigstens eines Betriebsparameters verwendet wird.

14. Verfahren nach einem der vorangehenden Ansprüche, **dadurch gekennzeichnet, dass** die Betriebsparameter die automatische Ausgabe wenigstens eines Workflow-Hinweises umfassen, insbesondere bezüglich eines zu verwendenden Hilfsmittels bei der Bildaufnahme und/oder zur Minimierung einer negativen Auswirkung auf den Patienten (14).

15. Bildaufnahmeeinrichtung (8), insbesondere Röntgeneinrichtung, aufweisend eine Aufnahmeanordnung und eine zur Durchführung eines Verfahrens nach einem der vorangehenden Ansprüche ausgebildete Steuereinrichtung (15).

16. Computerprogramm, welches die Schritte eines Verfahrens nach einem der Ansprüche 1 bis 14 durchführt, wenn es auf einer Steuereinrichtung (15) einer Bildaufnahmeeinrichtung (8) ausgeführt wird.

17. Elektronisch lesbarer Datenträger, auf dem ein Computerprogramm nach Anspruch 16 gespeichert ist.

## Geänderte Patentansprüche

### Geänderte Patentansprüche gemäss Regel 137(2) EPÜ.

1. Verfahren zum Betrieb einer medizinischen Bildaufnahmeeinrichtung (8) im Rahmen eines einem Bildaufnahmeziel dienenden Bildaufnahmevorgangs an einem Patienten (14), wobei die Steuerung der Bildaufnahmeeinrichtung (8) auf Grundlage von durch eine Steuereinrichtung (15) der Bildaufnahmeeinrichtung (8) umzusetzenden Betriebsparametern erfolgt, **dadurch gekennzeichnet, dass** die Betriebsparameter mittels eines Ermittlungsalgorithmus (6) vollständig automatisch aus wenigstens den Patienten (14) in Form eines Patientenmodells und/oder das Bildaufnahmeziel beschreibenden Eingangsdaten (2, 3, 4, 5) und einer Registrierung (1) des Patienten (14) mit einem Koordinatensystem der Bildaufnahmeeinrichtung (8) ermittelt und zur Steuerung der Bildaufnahmeeinrichtung (8) verwendet werden, wobei mittels der Registrierung (1) ein aufzunehmender Untersuchungsbereich des Patienten (14) und das Bildaufnahmeziel abgeleitet werden.

2. Verfahren nach Anspruch 1, **dadurch gekennzeichnet, dass** der Ermittlungsalgorithmus (6) wenigstens einen Algorithmus der künstlichen Intelligenz umfasst, der durch maschinelles Lernen trainiert wurde.

3. Verfahren nach Anspruch 2, **dadurch gekennzeichnet, dass** Trainingsdaten für den Algorithmus der künstlichen Intelligenz durch Protokollierung einer insbesondere wenigstens eine Einzelparameteranpassung umfassenden Tätigkeit eines Benutzers bei der Verwendung von vordefinierten Messprotokollen mit vordefinierten Betriebsparametern ermittelt werden.

4. Verfahren nach einem der vorangehenden Ansprüche, **dadurch gekennzeichnet, dass** die Betriebsparameter neben Bildaufnahmeparametern auch wenigstens einen Bildauswertungsparameter umfassen.

5. Verfahren nach einem der vorangehenden Ansprüche, **dadurch gekennzeichnet, dass** als den Patienten (14) beschreibende Patientendaten wenigstens das auf den aktuellen Patienten (14) angepasste Patientenmodell verwendet wird, welches lokal aufgelöst die Zusammensetzung des Patienten (14) beschreibt.

6. Verfahren nach einem der vorangehenden Ansprüche, **dadurch gekennzeichnet, dass** die Registrierung (1) unter Verwendung von Sensordaten wenigstens eines auf den Patienten (14) gerichteten Sensors und/oder von mit der Bildaufnahmeeinrichtung (8) aufgenommenen Bilddaten des Patienten (14) und/oder aufgrund von aus einem Informationssystem (18) abgerufenen Patientendaten und/oder aufgrund einer Ausgestaltung einer Patientenliege (13) der Bildaufnahmeeinrichtung (8) erhaltenen Patientenlagedaten erfolgt.

7. Verfahren nach Anspruch 6, **dadurch gekennzeichnet, dass** aufgrund bei der Registrierung (1) erhobener Registrierungsdaten das verwendete Patientenmodell an ein Koordinatensystem des aktuellen Patienten (14) angepasst wird, so dass in dem Patientenmodell enthaltene Merkmale in dem Koordinatensystem der Bildaufnahmeeinrichtung (8) lokalisiert werden können.

8. Verfahren nach einem der vorangehenden Ansprüche, **dadurch gekennzeichnet, dass** das Bildaufnahmeziel beschreibende Eingangsdaten (2, 3, 4, 5) aus einem Informationssystem (18) abgerufen werden und/oder aus anderen Eingangsdaten (2, 3, 4, 5) mittels des Ermittlungsalgorithmus abgeleitet werden, insbesondere aus einem innerhalb des Sichtfelds der Bildaufnahmeeinrichtung (8) positionierten Anteil des Patienten (14).

9. Verfahren nach einem der vorangehenden Ansprüche, **dadurch gekennzeichnet, dass** die Eingangsdaten (2, 3, 4, 5) auch wenigstens ein für den Patienten (14) bei dem Bildaufnahmevorgang verwendetes medizinisches Instrument beschreibende Instrumentendaten umfassen.

10. Verfahren nach Anspruch 9, **dadurch gekennzeichnet, dass** die Instrumentendaten unter Verwendung einer einen Informationsträger auf dem Instrument und/oder einer Verpackung des Instruments auslesenden Erkennungseinrichtung und/oder von Instrumentendaten eines Informationssystems (18) und/oder durch Auswertung von das Instrument zeigenden Bilddaten der Bildaufnahmeeinrichtung (8) ermittelt werden und/oder wenigstens eine für die Bildgebungsart der Bildaufnahmeeinrichtung (8) relevante Eigenschaft umfassen.

11. Verfahren nach einem der vorangehenden Ansprüche, **dadurch gekennzeichnet, dass** das Vorhandensein und/oder Positionen von Komponenten der Bildaufnahmeeinrichtung (8) aus der Stellung von diesen zugeordneten Aktoren und/oder durch den Komponenten zugeordnete Sensoren ermittelt werden.

12. Verfahren nach einem der vorangehenden Ansprüche, **dadurch gekennzeichnet, dass** die Eingangsdaten (2, 3, 4, 5) zyklisch und/oder bei Veränderungen aktualisiert werden, wobei eine Anpassung wenigstens eines der Betriebsparameter aufgrund der geänderten Eingangsdaten (2, 3, 4, 5) erfolgt.

13. Verfahren nach einem der vorangehenden Ansprüche, **dadurch gekennzeichnet, dass** durch den Ermittlungsalgorithmus (6) wenigstens eine für die verwendete Bildgebungsart der Bildaufnahmeeinrichtung (8) relevante Eigenschaft des Aufnahmebereichs des Patienten (14) ermittelt und zur Anpassung wenigstens eines Betriebsparameters verwendet wird.

14. Verfahren nach einem der vorangehenden Ansprüche, **dadurch gekennzeichnet, dass** die Betriebsparameter die automatische Ausgabe wenigstens eines Workflow-Hinweises an einen Benutzer umfassen, insbesondere bezüglich eines zu verwendenden Hilfsmittels bei der Bildaufnahme und/oder zur Minimierung einer negativen Auswirkung des Bildaufnahmevorgangs auf den Patienten (14).

15. Bildaufnahmeeinrichtung (8), insbesondere Röntgeneinrichtung, aufweisend eine Aufnahmeanordnung und eine zur Durchführung eines Verfahrens nach einem der vorangehenden Ansprüche ausgebildete Steuereinrichtung (15).

16. Computerprogramm, welches die Schritte eines Verfahrens nach einem der Ansprüche 1 bis 14 durchführt, wenn es auf einer Steuereinrichtung (15) einer Bildaufnahmeeinrichtung (8) ausgeführt wird.

17. Elektronisch lesbarer Datenträger, auf dem ein Computerprogramm nach Anspruch 16 gespeichert ist.
